# EUROPEAN PATENT APPLICATION

(11) **EP 2 537 544 A1**
(43) Date of publication of application: **26.12.2012**
(21) Application number: 11170826.9
(22) Date of filing: 21.06.2011
(51) Int. Cl.: A61M 5/00, B65D 83/02

(54) **Needle assembly storage device**

(71) Applicant: Sanofi-Aventis Deutschland GmbH, 65929 Frankfurt (DE)
(72) Inventor: The designation of the inventor has not yet been filed

(57) **Abstract**

Described is a needle assembly storage device (1) comprising an array (2) of needle assembly storage compartments (3) and a slider (4) movably disposed on the array (2). Each of the needle assembly storage compartments (3) includes an opening (3.1), rails (S1) disposed on opposing sides of the opening (3.1), and a latch (8) hingedly connected to the needle assembly storage compartment (3). The latch (8) has an open position (O) and a closed position (C) at least partially covering the opening (3.1). The slider (4) includes an opening (4.2) adapted to receive an injection device, arms adapted to engage the rails (S1) and a trailer (10). Movement of the slider (4) relative to the array (2) in a first direction causes the trailer (10) to move the latch (8) in the open position (O) to the closed position (C).

## Description

### Technical Field

The invention relates to a needle assembly storage device with an array of interconnected needle assembly storage compartments.

### Background of the Invention

Patients suffering from diseases like diabetes have to frequently self-administer injections. Injection devices like auto-injectors or pen injectors have been developed to facilitate self-administering injections. Typically, such injection devices are re-usable and refitted with sterile injection needle assemblies to minimize the risk of infections. However, such needle assemblies generally come packaged in a box, which is inconvenient for the patient to transport. To solve this problem, needle assembly storage devices were developed. A conventional needle assembly storage device contains a plurality of injection needle assemblies arranged in a portable container. Such conventional needle assembly storage devices are discussed in US 7,134,550, US 5,873,462 and WO 2009/136193.

However, there remains a need for needle assembly storage devices which provide for portability of needle assemblies in a convenient manner and allow for disposing of used needle assemblies.

### Summary of the Invention

It is an object of the present invention to provide a needle assembly storage device for storing and disposing of needle assemblies

The object is achieved by a needle assembly storage device according to claim 1. Preferred embodiments of the invention are given in the dependent claims.

In an exemplary embodiment, a needle assembly storage device comprises an array of needle assembly storage compartments and a slider movably disposed on the array. Each of the needle assembly storage compartments includes an opening, rails disposed on opposing sides of the opening, and a latch hingedly connected to the needle assembly storage compartment. The latch has an open position and a closed position at least partially covering the opening. The slider includes an opening adapted to receive an injection device, arms adapted to engage the rails and a trailer. Movement of the slider relative to the array in a first direction causes the trailer to move the latch in the open position to the closed position.

In an exemplary embodiment, a first end of the rails includes a notch. The slider may include a hook adapted to engage the notch when the opening of a given needle assembly storage compartment is aligned with the opening of the slider. The hook may include a bevelled edge adapted to engage a ramp on the notch when the slider is moved in the first direction and an abutment face adapted to engage the notch to prevent the slider from moving in a second direction opposite the first direction. At least one of an audible and a tactile feedback may be provided when the hook engages the notch.

In an exemplary embodiment, consecutive needle assembly storage compartments are connected by a hinge. The latch may include one or more projections adapted to engage a rim of the opening. The slider may include a guide adapted to align the injection device with the opening of the needle assembly storage compartment.

In another exemplary embodiment, a needle assembly storage device comprises an array of needle assembly storage compartments, a cover rotatably disposed on the array, and a slider coupled to the cover. Each of the needle assembly storage compartments includes an opening. The cover includes a plurality of cover elements disposed over the openings of the needle assembly storage compartments. The slider includes an opening adapted to align with the opening of the needle assembly storage compartment.

In an exemplary embodiment, the needle assembly storage compartments include guide rails formed on opposing sides of the opening or formed in sidewalls of the needle assembly storage compartments. The cover elements include stabilizers adapted to engage the guide rails. A given one of the needle assembly storage compartments includes one or more projections adapted to abut the slider or the cover to prevent movement of the slider or the cover.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: an isometric view of a needle assembly storage device according to an exemplary embodiment of the invention.
- Figure 2: an isometric view of a needle assembly storage compartment in an open position according to an exemplary embodiment of the invention.
- Figure 3: an isometric view of a needle assembly storage compartment with a flap in a closed position according to an exemplary embodiment of the invention.
- Figure 4: a sectional view a needle assembly storage device according to an exemplary embodiment of the invention.
- Figure 5: a sectional view a needle assembly storage device according to an exemplary embodiment of the invention.
- Figure 6: an isometric view of a slider according to an exemplary embodiment of the invention.
- Figure 7: an isometric view of a needle assembly storage device according to an exemplary embodiment of the invention.
- Figure 8: an isometric view of an array of needle assembly storage compartment according to an exemplary embodiment of the invention.
- Figure 9: an isometric view of a needle assembly storage compartment according to an exemplary embodiment of the invention.
- Figure 10: an isometric view of a slider according to an exemplary embodiment of the invention.
- Figure 11: an isometric view a needle assembly storage device according to an exemplary embodiment of the invention.

Corresponding parts are marked with the same reference symbols in all figures.

### Detailed Description

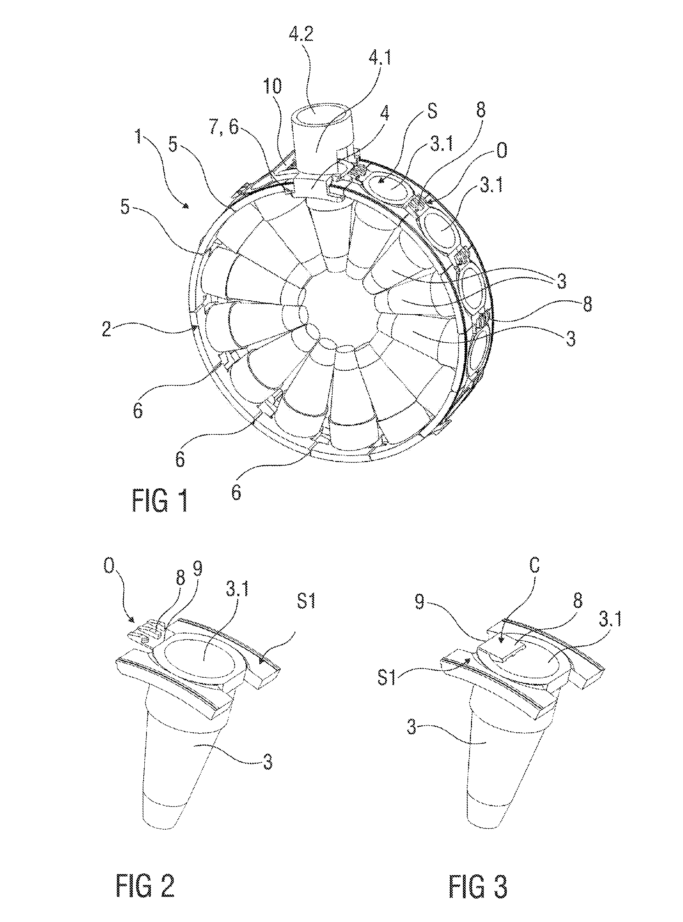

Figure 1 shows an exemplary embodiment of a needle assembly storage device 1 according to the present invention. In an exemplary embodiment, the needle assembly storage device 1 comprises an array 2 of needle assembly storage compartments 3 in which consecutive needle assembly storage compartments 3 are connected. The array 2 may be manufactured from a flexible plastic material by a method of injection moulding. The array 2 may be used in an elongate shape or formed into a rounded, circular, or ellipsoidal shape by bending the array 2 and connecting (temporarily or permanently) terminal ends. The array 2, or portions thereof, may be formed from a material class capable of resisting high strain including, but not limited to, polyamide, polyethylene and polypropylene.

In an exemplary embodiment, as shown in Figures 2 and 3, a needle assembly storage compartment 3 is adapted to contain a needle assembly (not shown) in a sterile environment. A needle assembly generally comprises a needle hub adapted to engage an injection device and a needle (e.g., a double-tipped needle) disposed in the needle hub. The exemplary embodiment of the needle assembly storage compartment 3 has a form which is generally adapted to accommodate a shape of the needle assembly. For example, the needle assembly storage compartment 3 may have a conical or frusto-conical shape. An opening 3.1 may be formed at a proximal end of the needle assembly storage compartment 3 for allowing an injection device to engage and remove the needle assembly and disengage and deposit the needle assembly in the needle assembly storage compartment 3. In an exemplary embodiment, the opening 3.1 may be sealed with a peelable film. In another exemplary embodiment, a tape may disposed around the array 1, sealing all of the openings 3.1.

A rail S1 may be disposed adjacent the opening 3.1. In an exemplary embodiment, two rails S1 are disposed in parallel on opposing sides of the opening 3.1. Consecutive needle assembly storage compartments 3 may be linked by the respective rails S1. For example, a hinge 5 may be formed between consecutive rails S1 to allow for relative motion (e.g., bending) in at least one plane. A notch 6 is formed between consecutive needle assembly storage compartments 3 under the hinge 5.

In an exemplary embodiment, a latch 8 is connected to the needle assembly storage compartment 3 by a hinge 9. The latch 8 may be moved between an open position (Figure 2) and a closed position C (Figure 3). In the closed position C, the latch 8 may at least partially cover the opening 3.1 of the needle assembly storage compartment 3, preventing removal of the needle assembly therein. The latch 8 may have one or more projections which engage a rim of the opening 3 of the needle assembly storage compartment 3 when in the closed position C. For example, the projections may frictionally engage the rim or engage slots formed in the rim. In an exemplary embodiment, the latch 8 may be locked in the closed position C.

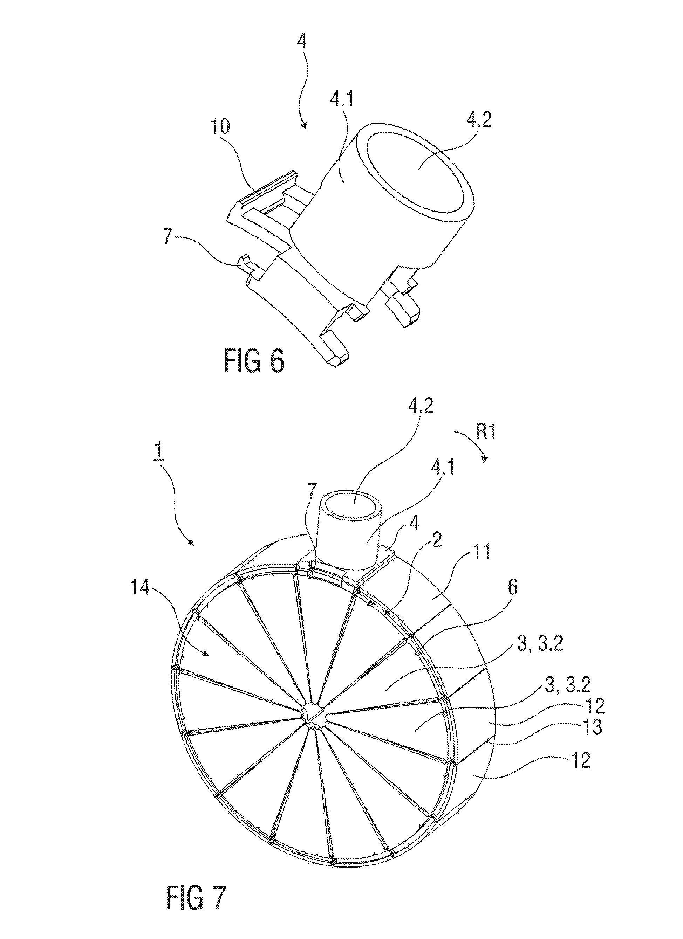

Referring back to the exemplary embodiment show in Figure 1, a slider 4 is movably disposed on the array 1. In an exemplary embodiment shown in Figure 6, the slider 4 may include a guide 4.1 adapted to receive an injection device, e.g., a pen injector, an auto-injector, a pre-filled syringe, etc. For example, the guide 4.1 may be cylindrical and have a diameter substantially equal to a diameter of the injection device to ensure that the injection device is properly aligned with the needle assembly in the needle assembly storage compartment 2 when engaging/disengaging. The guide 4.1 may include an opening 4.2 for receiving the injection device.

In an exemplary embodiment, the device 1 may be comprised of two parts, the array 2 and the slider 4.

In an exemplary embodiment, the slider 4 includes a pair of arms on opposing sides of the guide 4.1 which engage the rails S1 on the needle assembly storage compartments 3. The engagement of the arms and the rails S1, allows the slider 4 to translate along the array 2.

In an exemplary embodiment, each arm on the slider 4 includes a hook 7 to engage the notch 6 between consecutive needle assembly storage compartments 3. In an exemplary embodiment, the hook 7 engages the notch 6 when the guide 4.1 of the slider 4 is aligned with the opening 3.1 of the needle assembly storage compartment 3. The hook 7 may include a bevelled edge for disengaging the notch 6 when the slider 4 is moved in a first direction R1 and an abutment face for abutting the notch 6 when the slider 4 is moved in a second direction R2. The notch 6 may have a ramp to engage the bevelled edge of the hook 7.

In an exemplary embodiment, the slider 4 includes a trailer 10. As explained further below, the trailer 10 may be disposed on a base of the slider 4 and ride along the outer surface of the needle assembly storage compartments 3 to engage the latches 8 as the slider 4 is moved along the array 2.

As will be described further below, access to the needle assemblies in the respective needle assembly storage compartments 3 may be gained by translating the slider 4 around an outer circumference S of the array 2

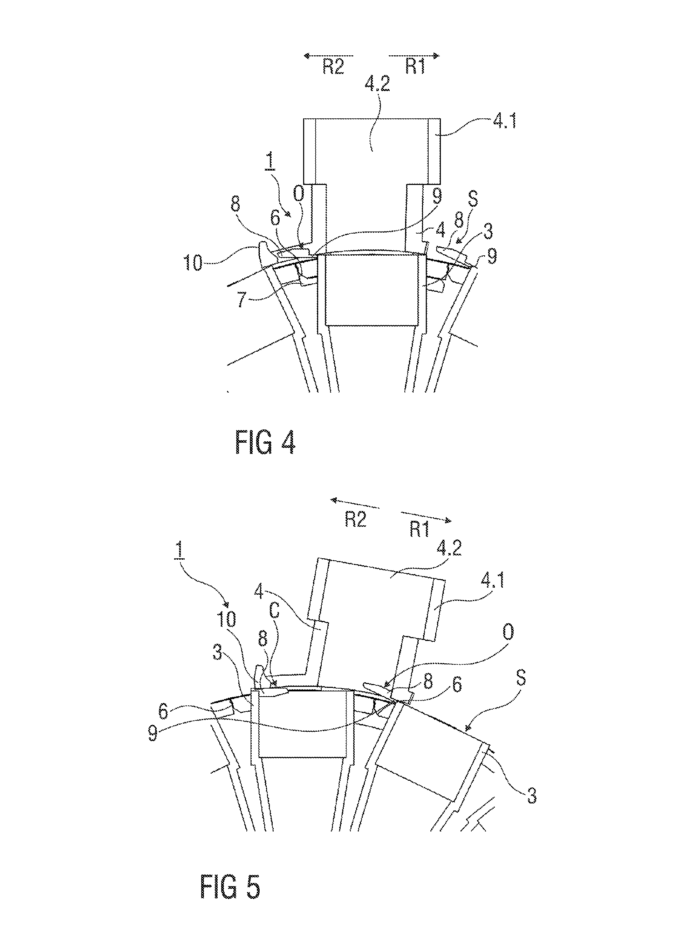

As shown in the exemplary embodiment in Figure 4, the slider 4 is disposed over a needle assembly storage compartment 3 such that the guide 4.1 of the slider 4 is aligned with the opening of the needle assembly storage compartment 3. The injection device may be inserted into the guide 4.1 to engage the needle assembly in the needle assembly storage compartment 3. By engagement of the hook 7 in the notch 6, the slider 4 may be prevented from moving while the injection device is engaging (or disengaging) the needle assembly.

After a used needle assembly is deposited in the needle assembly storage compartment 3, the slider 4 may be advanced along the array 2 to cover that needle assembly storage compartment 3 to prevent re-use of the needle assembly. As the slider 4 is moved along the array 2 in a first direction the trailer 10 engages the latch 8 on the needle assembly storage compartment 3 (in the open position O, as shown in Figure 4). Further movement of the slider in the first direction causes the trailer 10 to transition the latch 8 into the closed position C, as shown in Figure 5. A tactile and/or audible feedback is provided when the hook 7 engages the next notch 6 in the array 2.

In an exemplary embodiment, the needle assembly storage device 1 may comprise an outer housing (not shown) that encompasses the array 2 of needle assembly storage compartments 3. The array 2 may be rotatably supported in the outer housing, or the outer housing (with the slider 4 coupled thereto or formed integrally therewith) may be rotatable relative to the array 2.

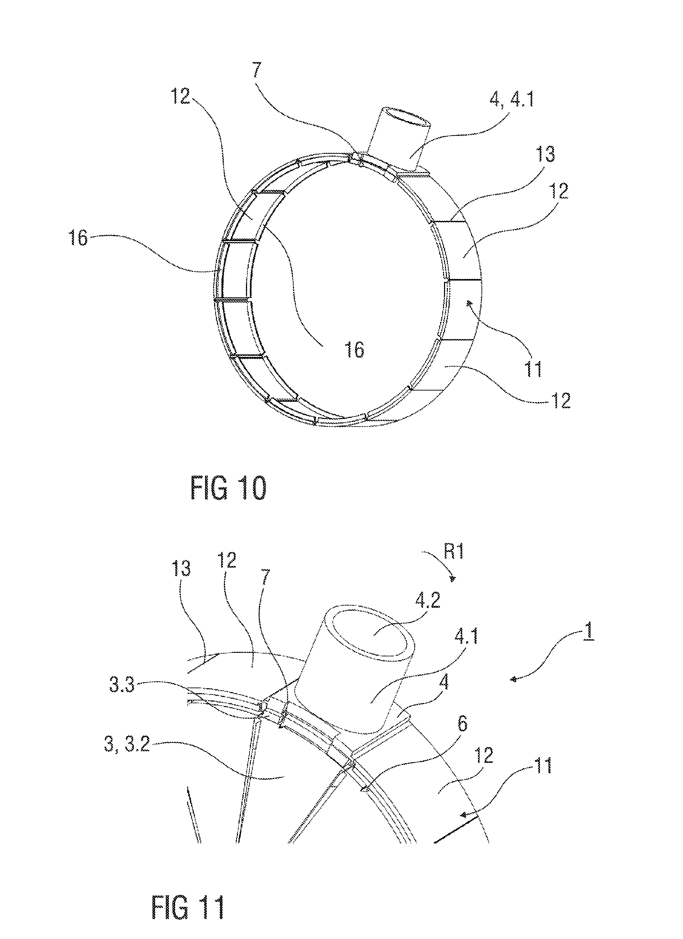

Figures 7 to 11 show another exemplary embodiment of a needle assembly storage device 1 according to the present invention. In this exemplary embodiment (shown in Figure 7), the needle assembly storage device 1 includes a cover 11 that covers the outer surface of the array 2 of the needle assembly storage compartments 3. The cover 11 may be formed by a plurality of cover elements 12. Consecutive cover elements 12 may be connected by a hinge. In an exemplary embodiment in which the array 2 is formed in a circular or ellipsoidal shape, a first terminal end of the cover 11 may be attached to a first edge of the slider 4 and a second terminal end of the cover 11 may be attached to a second edge of the slider 4. Thus, as the slider 4 is moved over the array 2, the cover 11 is moved over the array 2, as well. As shown in Figure 10, in an exemplary embodiment, stabilizers 16 may be formed on an inner surface of each of the cover elements 12. The stabilizers 16 may be elongate projections parallel to each other and to lateral edges of the cover element 12.

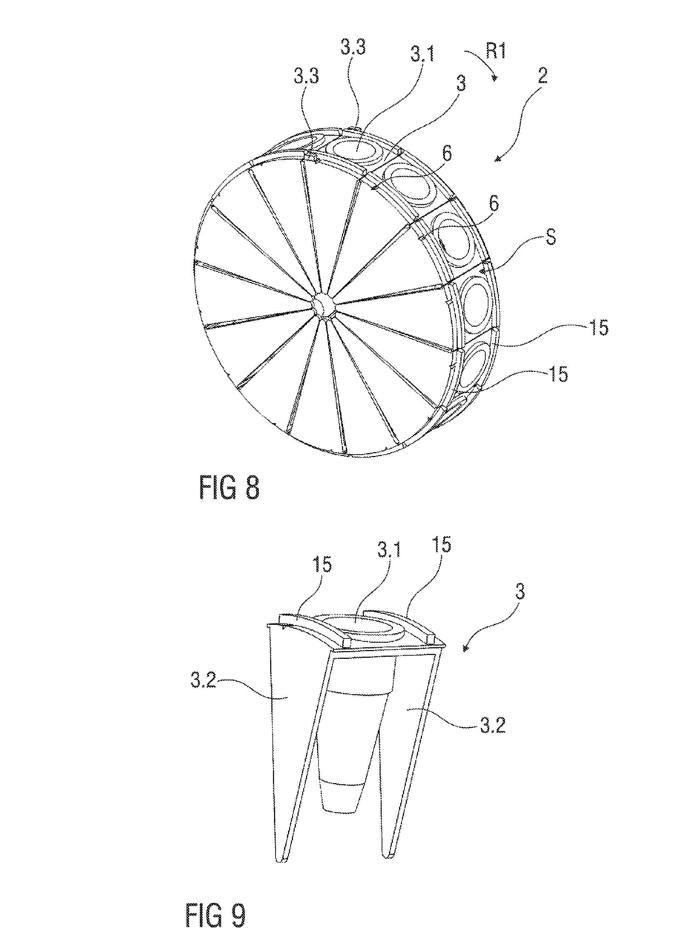

As shown in Figures 8 and 9, an exemplary embodiment of the needle assembly storage compartment 3 has a form which is generally adapted to accommodate a shape of the needle assembly. For example, the needle assembly storage compartment 3 may have a conical or frusto-conical shape. An opening 3.1 may be formed at a proximal end of the needle assembly storage compartment 3 for allowing an injection device to engage and remove the needle assembly and disengage and deposit the needle assembly in the needle assembly storage compartment 3.

In an exemplary embodiment, adjacent to and on opposing sides of the opening 3.1, guide rails 15 may be formed. While the guide rails 15 are depicted as being disposed in the same plane as the opening 3.1, those of skill in the art will understand the guide rails 15 may be project from (or be formed in) sides of the needle assembly storage compartment 3, and there may be one or more guide rails 15. When the device 1 is assembled, the stabilizers 16 of the cover elements 12 may abut the guide rails 12 (e.g., the stabilizers 16 may be lateral to the guide rails 12), for example, to prevent lateral displacement of the cover 11 relative to the array 2.

Figure 9 shows an exemplary embodiment of a needle assembly storage compartment 3. The needle assembly storage compartment 3 may include sidewalls 32 extending along and covering a length of the needle assembly storage compartment 3. In an exemplary embodiment, an outer surface of the sidewalls 32 may be textured for facilitating gripping of the device 1 when the slider 4 and/or the cover 11 is being rotated.

Referring to Figure 8, in an exemplary embodiment, one of the needle assembly storage compartments 3 may have one or more projections 3.3 formed thereon. The projections 3.3 may extend laterally from lateral edges of the one needle assembly storage compartment 3. As shown in Figure 11, in an exemplary embodiment, the slider 4 is initially positioned over the one needle assembly storage compartment 3 with the one or more projections 3.3. The slider 4 and/or the cover 11 may include a stop to abut the projections 3.3 when the slider 4 and/or the cover 11 has been rotated over all of the needle assembly storage compartments 3 and all of the needle assemblies have been used. Abutment of the stop and the projections 3.3 may prevent reuse of used needle assemblies. In an exemplary embodiment, the stop may lockingly engage the projections 3.3 such that rotation of the slider 4 and/or the cover 11 is prevented after the stop has engaged the projections 3.3.

### List of References

- 1: needle assembly storage device
- 2: array
- 3: needle assembly storage compartment
- 3.1: opening
- 3.2: side wall
- 3.3: projection
- 4: slider
- 4.1: guide
- 4.2: opening
- 5: hinge
- 6: notch
- 7: hook
- 8: latch
- 9: hinge
- 10: trailer
- 11: cover
- 12: cover element
- 13: third hinge
- 14: outer housing
- 15: guide rail
- 16: stabilizer

- S: outer circumference
- S1: rails
- O: open position
- C: closed position
- R1: first direction
- R2: second direction

## Claims

1. A needle assembly storage device (1) comprising:
an array (2) of needle assembly storage compartments (3), each of the needle assembly storage compartments (3) including an opening (3.1), rails (S1) disposed on opposing sides of the opening (3.1), and a latch (8) hingedly connected to the needle assembly storage compartment (3) and having an open position (O) and a closed position (C) at least partially covering the opening (3.1); and
a slider (4) movably disposed on the array (2), wherein the slider (4) includes an opening (4.2) adapted to receive an injection device, arms adapted to engage the rails (S1) and a trailer (10),
wherein movement of the slider (4) relative to the array (2) in a first direction causes the trailer (10) to move the latch (8) in the open position (O) to the closed position (C).

2. The needle assembly storage device (1) according to claim 1, wherein a first end of the rails (S1) includes a notch (6).

3. The needle assembly storage device (1) according to claim 2, wherein the slider (4) includes a hook (7) adapted to engage the notch (6) when the opening (3.1) of a given needle assembly storage compartment (3) is aligned with the opening (4.2) of the slider (4).

4. The needle assembly storage device (1) according to claim 3, wherein the hook (7) includes a bevelled edge adapted to engage a ramp on the notch (6) when the slider (4) is moved in the first direction and an abutment face adapted to engage the notch (6) to prevent the slider (4) from moving in a second direction opposite the first direction.

5. The needle assembly storage device (1) according to claim 3, wherein at least one of an audible and a tactile feedback is provided when the hook (7) engages the notch (6).

6. The needle assembly storage device (1) according to any one of the preceding claims, wherein consecutive needle assembly storage compartments (3) are connected by a hinge (5).

7. The needle assembly storage device (1) according to any one of the preceding claims, wherein the latch (8) includes one or more projections adapted to engage a rim of the opening (3.1).

8. The needle assembly storage device (1) according to any one of the preceding claims, wherein the slider (4) includes a guide (4.1) adapted to align the injection device with the opening (3.1) of the needle assembly storage compartment (3).

9. A needle assembly storage device (1) comprising:
an array (2) of needle assembly storage compartments (3), each of the needle assembly storage compartments (3) including an opening (3.1)
a cover (11) rotatably disposed on the array (2), the cover (11) including a plurality of cover elements (12) disposed over the openings (3.1) of the needle assembly storage compartments (3);
a slider (4) coupled to the cover (11), the slider (4) including an opening (4.2) adapted to align with the opening (3.1) of the needle assembly storage compartment (3).

10. The needle assembly storage device (1) according to claim 9, wherein the needle assembly storage compartments (3) include guide rails (15) formed on opposing sides of the opening (3.1) or formed in sidewalls (3.2) of the needle assembly storage compartments (3).

11. The needle assembly storage device (1) according to claim 10, wherein the cover elements (12) include stabilizers (16) adapted to engage the guide rails (15).

12. The needle assembly storage device (1) according to claim 9, wherein a given one of the needle assembly storage compartments (3) includes one or more projections (3.3) adapted to abut the slider (4) or the cover (11) to prevent movement of the slider (4) or the cover (11).
